(19) Europäisches Patentamt
European Patent Office
Office européen des brevets

(11) Veröffentlichungsnummer: **0 157 206**
**A2**

(12) # EUROPÄISCHE PATENTANMELDUNG

(21) Anmeldenummer: **85102518.9**

(22) Anmeldetag: **06.03.85**

(51) Int. Cl.⁴: **C 07 D 313/08**, C 07 D 311/76, A 61 K 31/335, C 07 D 407/12

(30) Priorität: **16.03.84 DE 3409612**

(43) Veröffentlichungstag der Anmeldung: **09.10.85 Patentblatt 85/41**

(84) Benannte Vertragsstaaten: **AT BE CH DE FR GB IT LI LU NL SE**

(71) Anmelder: **BOEHRINGER INGELHEIM KG, D-6507 Ingelheim am Rhein (DE)**

(72) Erfinder: **Abele, Wolfgang, Dr., Kirchstrasse 2a, D-6531 Waldalgesheim (DE)**
Erfinder: **Köppe, Herbert, Dr., Neuweg 72, D-6507 Ingelheim/Rhein (DE)**
Erfinder: **Esser, Franz, Dr., Posener Strasse 30, D-6507 Ingelheim/Rhein (DE)**
Erfinder: **Gaida, Wolfram, Dr., Paul-Clemen-Strasse 14, D-6507 Ingelheim/Rhein (DE)**
Erfinder: **Hoefke, Wolfgang, Dr., Rosselstrasse 21, D-6200 Wiesbaden (DE)**
Erfinder: **Streller, Ilse, Dr., Waldstrasse 16, D-6534 Stromberg (DE)**
Erfinder: **Reichl, Richard, Dr., Im Hippel 55, D-6535 Gau-Algesheim (DE)**
Erfinder: **Traunecker, Werner, Dr., Birkenweg 1, D-6531 Münster-Sarmsheim (DE)**

(54) **Neue substituierte Isochromane und Oxepine, deren Säureadditionssalze, diese enthaltende Arzneimittel und Verfahren zu deren Herstellung.**

(57) Die Erfindung betrifft Isochromane und Oxepine der allgemeinen Formel (I)

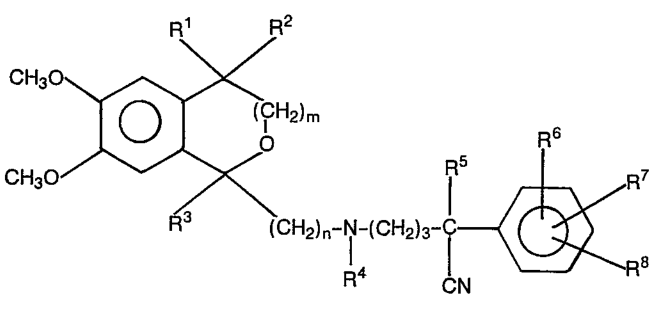

worin
$R^1$ ein Wasserstoffatom oder eine gerade oder verzweigte Alkylgruppe mit bis zu 4 C-Atomen,
$R^2$ ein Wasserstoffatom oder eine gerade oder verzweigte Alkylgruppe mit bis zu 4 C-Atomen oder zusammen mit $R^1$ eine Alkylengruppe mit 3 bis 5 C-Atomen,
$R^3$ ein Wasserstoffatom oder eine gerade oder verzweigte Alkylgruppe mit bis zu 4 C-Atomen,
$R^4$ ein Wasserstoffatom oder eine gerade oder verzweigte Alkylgruppe mit bis zu 4 C-Atomen,
$R^5$ ein Wasserstoffatom oder eine gerade oder verzweigte Alkylgruppe mit 1 bis 6 C-Atomen oder eine Pyridinylgruppe,
$R^6$ ein Wasserstoffatom, ein Fluor-, Chlor- oder Bromatom, eine gerade oder verzweigte Alkylgruppe mit 1 bis 6 C-Atomen, eine Hydroxi- oder Alkoxigruppe mit 1 bis 4 C-Atomen oder eine Trifluormethylgruppe,
$R^7$ ein Wasserstoffatom, ein Fluor-, Chlor- oder Bromatom, eine gerade oder verzweigte Alkylgruppe mit 1 bis 6 C-Atomen, eine Hydroxi- oder Alkoxigruppe mit 1 bis 4 C-Atomen oder zusammen mit $R^6$ eine Methylendioxigruppe,
$R^8$ ein Wasserstoffatom, eine Hydroxi- oder Alkoxigruppe mit 1 bis 4 C-Atomen,
m die Zahlen 1 oder 2 und
n die Zahlen 1, 2 oder 3 bedeuten, und deren Säureadditionssalze.

Die Verbindungen wirken calciumantagonistisch und antihypertonisch.

Gegenstand der Erfindung sind neue substituierte Isochromane und Oxepine der allgemeinen Formel (I)

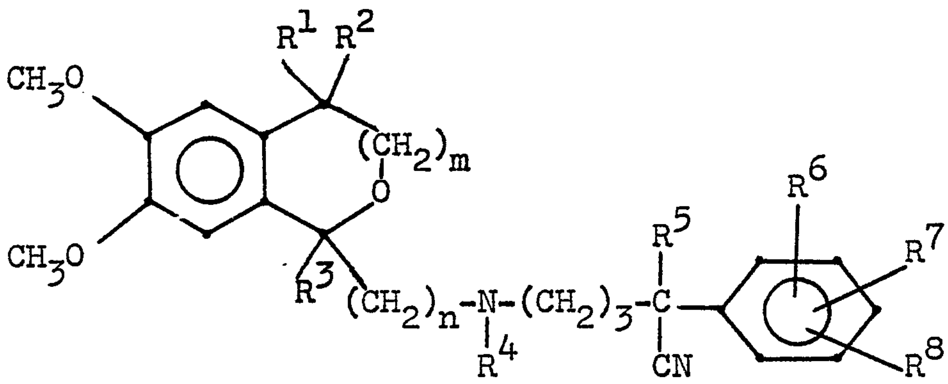

I

sowie deren Säureadditionssalze mit wertvollen therapeutischen Eigenschaften.

In der Formel (I) bedeutet

$R^1$ ein Wasserstoffatom oder eine gerade oder verzweigte Alkylgruppe mit bis zu 4 C-Atomen,

$R^2$ ein Wasserstoffatom oder eine gerade oder verzweigte Alkylgruppe mit bis zu 4 C-Atomen oder zusammen mit $R^1$ eine Alkylengruppe mit 3 bis 5 C-Atomen,

$R^3$ ein Wasserstoffatom oder eine gerade oder verzweigte Alkylgruppe mit bis zu 4 C-Atomen,

$R^4$ ein Wasserstoffatom oder eine gerade oder verzweigte Alkylgruppe mit bis zu 4 C-Atomen,

$R^5$ ein Wasserstoffatom oder eine gerade oder verzweigte Alkylgruppe mit 1 bis 6 C-Atomen oder eine Pyridinylgruppe,

$R^6$ ein Wasserstoffatom, ein Fluor-, Chlor- oder Bromatom, eine gerade oder verzweigte Alkylgruppe mit 1 bis 6 C-Atomen, eine Hydroxi- oder Alkoxigruppe mit 1 bis 4 C-Atomen oder eine Trifluormethylgruppe,

$R^7$ ein Wasserstoffatom, ein Fluor-, Chlor- oder Bromatom, eine gerade oder verzweigte Alkylgruppe mit 1 bis 6 C-Atomen, eine Hydroxi- oder Alkoxigruppe mit 1 bis 4 C-Atomen oder zusammen mit $R^6$ eine Methylen-dioxigruppe,

$R^8$ ein Wasserstoffatom, ein Fluor-, Chlor- oder Bromatom, eine Hydroxi- oder Alkoxigruppe mit 1 bis 4 C-Atomen,

m die Zahlen 1 oder 2

n die Zahlen 1, 2 oder 3.

Bevorzugt sind Isochromane und Oxepine der allgemeinen Formel (Ia)

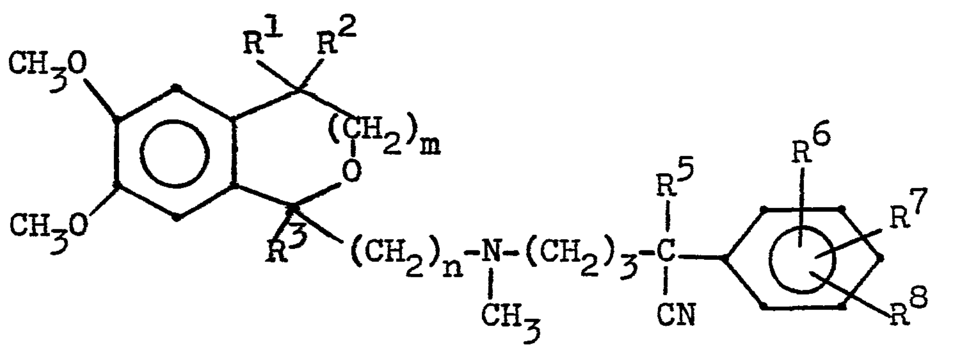

Ia

worin $R^1$, $R^2$ und $R^3$, die gleich oder verschieden sein können, ein Wasserstoffatom oder eine Methylgruppe,
$R^5$ eine Isopropyl- oder Pyridinylgruppe,
$R^6$ ein Wasserstoff-, Fluor- oder Chloratom, eine Trifluormethyl- oder Methoxigruppe,

$R^7$ ein Wasserstoff-, Fluor- oder Chloratom, eine tertiär-Butylgruppe, eine Methoxigruppe oder zusammen mit $R^6$ eine Methylendioxigruppe,

$R^8$ ein Wasserstoffatom oder eine Methoxigruppe,

m die Zahlen 1 oder 2 und

n die Zahlen 1 oder 2 bedeuten sowie deren Säureadditionssalze.

Ganz besonders bevorzugt sind Verbindungen der allgemeinen Formel (Ib)

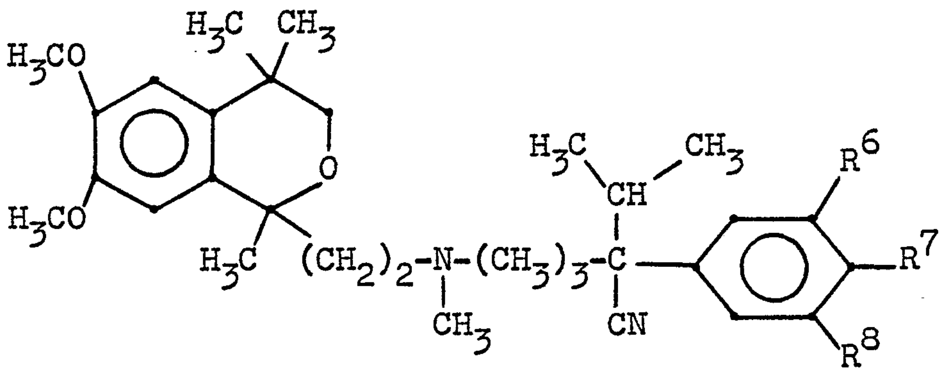

Ib

worin

$R^6$ ein Wasserstoff- oder Chloratom oder eine Methoxigruppe,

$R^7$ ein Fluor- oder Chloratom oder eine Methoxigruppe und

$R^8$ ein Wasserstoffatom oder eine Methoxigruppe bedeuten sowie deren Säureadditionssalze.

Ein weiterer Gegenstand der Erfindung betrifft die Herstellung der neuen Verbindungen. Das Herstellungsverfahren ist dadurch gekennzeichnet, daß man ein Isochroman oder Oxepinderivat der allgemeinen Formel (II)

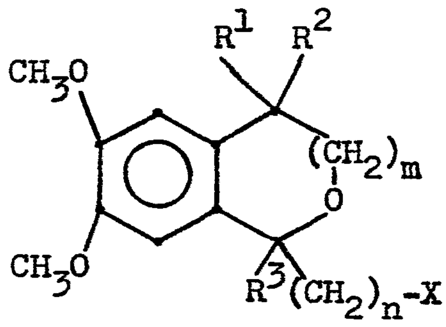

II

worin
$R^1$, $R^2$, $R^3$, m und n wie oben angegeben definiert sind und X ein Chlor-, Brom- oder Jodatom oder eine Alkylsulfonyloxi- oder Arylsulfonyloxigruppe bedeutet, mit einem Phenyl-aminopropyl-acetonitril der allgemeinen Formel (III)

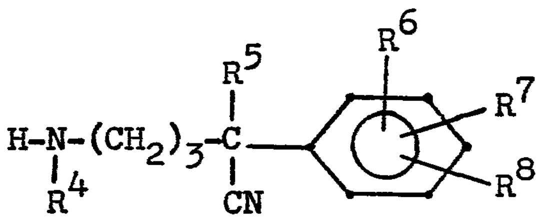

III

worin
$R^4$, $R^5$, $R^6$, $R^7$ und $R^8$ die oben angegebene Bedeutung besitzen, umsetzt und die erhaltene Verbindung gewünschtenfalls in ein Säureadditionssalz überführt.

Die Umsetzung wird bevorzugt in einem aprotischen Lösungsmittel,wie z.B. Toluol oder in einem aprotischen polaren Lösungsmittel, wie z.B. Dimethylformamid unter Zusatz eines säurebindenden Mittels, z.B. einer anorganischen Base, wie Natriumcarbonat, Natronlauge oder einer organischen Base, wie Triethylamin durchgeführt. Die Reaktionstemperatur kann von Raumtemperatur bis 160°C gewählt werden. Bevorzugt wird die Reaktion zwischen 60 und 120°C durchgeführt.

Die Isochromane und Oxepine der allgemeinen Formel I, Ia und Ib besitzen ein asymmetrisches Kohlenstoffatom und lassen sich in bekannter Weise in ihre Antipoden trennen. Die Antipoden erhält man auch, wenn man von optisch aktiven Ausgangsstoffen ausgeht.

Die erfindungsgemäßen Isochromane und Oxepine der allgemeinen Formel I können auf übliche Weise in ihre Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxibenzoesäure, p-Aminobenzoesäure, Phthalsäure, Zimtsäure, Salicylsäure, Ascorbinsäure, Methansulfonsäure, Ethanphosphonsäure.

Die Verbindungen der allgemeinen Formel I haben an isolierten Organen und im Tierversuch wertvolle therapeutische, insbesondere calciumantagonistische Eigenschaften gezeigt, die sich in Blutdrucksenkung, Bradycardie, negativer Inotropie und Erweiterung coronarer und peripherer Gefäße ausdrücken.

Die neuen Verbindungen können daher beispielsweise zur Behandlung oder Prophylaxe coronarer Herzkrankheiten und des Bluthochdrucks in der Medizin eingesetzt werden.

Die erfindungsgemäßen Verbindungen der allgemeinen Formel I können allein oder in Kombination mit anderen Wirkstoffen zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können durch Mischen des oder der Wirkstoffe mit bekannten Hilfsstoffen, beispielsweise inerten Verdünnungsmitteln wie

Calciumcarbonat, Calciumphosphat oder Milchzucker; Sprengmitteln wie Maisstärke, Alginsäure; Bindemitteln wie Stärke oder Gelatine; Schmiermitteln wie Magnesiumstearat oder Talkum und/oder Mitteln zur Erzielung eines Depoteffektes wie Carboxipolymethylen, Carboximethylcellulose, Celluloseacetatphthalat oder Polyvinylacetat erhalten werden.

Die neuen Verbindungen der allgemeinen Formel I können enteral oder parenteral angewandt werden. Bei oraler Verabreichung haben sich Einzeldosierungen von 5 bis 20 mg, vorzugsweise 10 bis 15 mg als zweckmäßig erwiesen.

In der folgenden Tabelle 1 sind die erfindungsgemäßen Verbindungen der allgemeinen Formel I mit ihren physikalischen Kenndaten zusammengestellt.

Tabelle 1: Erfindungsgemäße Verbindungen der Formel I

| Nr. | $R^1$ | $R^2$ | $R^3$ | $R^4$ | $R^5$ | $R^6$ | $R^7$ | $R^8$ | m | n | Rf-Werte |
|---|---|---|---|---|---|---|---|---|---|---|---|
| 1 | H | H | H | $CH_3$ | $CH(CH_3)_2$ | $(3)OCH_3$ | $(4)OCH_3$ | H | 1 | 1 | 0.47 a) |
| 2 | H | H | H | $CH_3$ | $CH(CH_3)_2$ | H | (4) F | H | 1 | 1 | 0.48 b) |
| 3 | H | H | H | $CH_3$ | $CH(CH_3)_2$ | $(3)CF_3$ | H | H | 1 | 1 | 0.55 a) |
| 4 | H | H | H | $CH_3$ | $CH(CH_3)_2$ | $(3)OCH_3$ | $(4)OCH_3$ | $(5)OCH_3$ | 1 | 1 | 0.48 a) |
| 5 | H | H | H | $CH_3$ | $CH(CH_3)_2$ | $(3)CF_3$ | H | H | 2 | 1 | 0.61 a) |
| 6 | H | H | H | $CH_3$ | $CH(CH_3)_2$ | (2) F | $(3)OCH_3$ | $(4)OCH_3$ | 2 | 1 | 0.64 a) |
| 7 | H | H | H | $CH_3$ | $CH(CH_3)_2$ | (2) F | $(3)OCH_3$ | $(4)OCH_3$ | 1 | 1 | 0.51 a) |
| 8 | $CH_3$ | $CH_3$ | H | $CH_3$ | $CH(CH_3)_2$ | $(3)CF_3$ | H | H | 1 | 2 | 0.43 a) |
| 9 | H | H | H | $CH_3$ | $CH(CH_3)_2$ | $(3)CF_3$ | H | H | 1 | 1 | 0.39 a) |
| 10 | H | H | $CH_3$ | $CH_3$ | $CH(CH_3)_2$ | H | (4) F | H | 1 | 2 | 0.32 a) |
| 11 | $CH_3$ | $CH_3$ | H | $CH_3$ | $CH(CH_3)_2$ | (3) Cl | (4) Cl | H | 1 | 2 | 0.38 a) |
| 12 | H | H | $CH_3$ | $CH_3$ | $CH(CH_3)_2$ | (3) Cl | (4) Cl | H | 1 | 2 | 0.22 a) |
| 13 | H | H | H | $CH_3$ | $2\text{-}C_5H_4N$ | $(3)OCH_3$ | $(4)OCH_3$ | H | 1 | 1 | 0.38 a) |
| 14 | H | H | H | $CH_3$ | $CH(CH_3)_2$ | (3) Cl | (4) Cl | H | 1 | 1 | 0.54 a) |
| 15 | H | H | H | $CH_3$ | $CH(CH_3)_2$ | (3) Cl | (4) Cl | H | 2 | 1 | 0.55 a) |
| 16 | H | H | H | $CH_3$ | $2\text{-}C_5H_4N$ | (3) $CF_3$ | H | H | 1 | 1 | 0.59 c) |
| 17 | $CH_3$ | $CH_3$ | H | $CH_3$ | $CH(CH_3)_2$ | H | $(4)C(CH_3)_3$ | H | 1 | 2 | 0.32 d) |
| 18 | H | H | H | $CH_3$ | $CH(CH_3)_2$ | H | $(4)C(CH_3)_3$ | H | 1 | 1 | 0.33 d) |
| 19 | $CH_3$ | $CH_3$ | H | $CH_3$ | $CH(CH_3)_2$ | H | (4) F | H | 1 | 2 | 0.52 c) |
| 20 | H | H | $CH_3$ | $CH_3$ | $CH(CH_3)_2$ | $(3)OCH_3$ | $(4)OCH_3$ | $(5)OCH_3$ | 1 | 2 | 0.27 d) |
| 21 | H | H | $CH_3$ | $CH_3$ | $CH(CH_3)_2$ | $(3,4)-O-CH_2-O-$ | | H | 1 | 2 | 0.27 d) |
| 22 | $CH_3$ | $CH_3$ | H | $CH_3$ | $2\text{-}C_5H_4N$ | H | (4) F | H | 1 | 2 | 0.13 d) |
| 23 | $CH_3$ | $CH_3$ | $CH_3$ | $CH_3$ | $CH(CH_3)_2$ | (3) Cl | (4) Cl | H | 1 | 2 | 0.29 d) |
| 24 | H | H | H | $CH_3$ | $CH(CH_3)_2$ | H | (4) F | H | 1 | 2 | 0.43 a) |
| 25 | H | H | $CH_3$ | $CH_3$ | $CH(CH_3)_2$ | H | $(4)C(CH_3)_3$ | H | 1 | 2 | 0.51 a) |
| 26 | H | H | H | $CH_3$ | $CH(CH_3)_2$ | $(3)OCH_3$ | $(4)OCH_3$ | $(5)OCH_3$ | 1 | 2 | 0.42 a) |
| 27 | H | H | H | $CH_3$ | $CH(CH_3)_2$ | $(3)OCH_3$ | $(4)OCH_3$ | H | 1 | 2 | 0.44 a) |
| 28 | $CH_3$ | H | H | $CH_3$ | $CH(CH_3)_2$ | $(3,4)-O-CH_2-O-$ | | H | 1 | 2 | 0.41 a) |
| 29 | $CH_3$ | $CH_3$ | $CH_3$ | $CH_3$ | $CH(CH_3)_2$ | $(3)OCH_3$ | $(4)OCH_3$ | $(5)OCH_3$ | 1 | 2 | 0.34 a) |
| 30 | H | H | H | $CH_3$ | $CH(CH_3)_2$ | (3) Cl | (4) Cl | H | 1 | 2 | 0.30 a) |
| 31 | $CH_3$ | $CH_3$ | $CH_3$ | $CH_3$ | $CH(CH_3)_2$ | H | (4) F | H | 1 | 2 | 0.36 a) |
| 32 | $CH_3$ | $CH_3$ | H | $CH_3$ | $CH(CH_3)_2$ | $(3)OCH_3$ | $(4)OCH_3$ | H | 1 | 2 | 0.39 a) |
| 33 | H | H | H | $CH_3$ | $CH(CH_3)_2$ | $(3)CF_3$ | H | H | 1 | 2 | 0.42 a) |
| 34 | $CH_3$ | $CH_3$ | H | $CH_3$ | $CH(CH_3)_2$ | $(3)OCH_3$ | $(4)OCH_3$ | $(5)OCH_3$ | 1 | 2 | 0.43 a) |
| 35 | H | H | $CH_3$ | $CH_3$ | $CH(CH_3)_2$ | $(3)OCH_3$ | $(4)OCH_3$ | H | 1 | 2 | 0.21 a) |

Elutionsmittelgemische:

a) $CH_2Cl_2/CH_3OH$ 9:1; b) $CH_2Cl_2/CH_3OH$ 95:5;

c) $CH_2Cl_2/CH_3OH$ 8:2; d) $CH_2Cl_2/CH_3CN/C_2H_5OH$ 7:2:1

## Herstellungsbeispiele

### Beispiel 1

**6,7-Dimethoxi-1-{1-[N-(4-cyano-5-methyl-4-(m-trifluoromethyl-phenyl)-hexyl)-N-methyl-amino]-methyl}-isochroman (Tabelle 1, Verbindung Nr. 3)**

2 g 1-Brommethyl-6,7-dimethoxi-isochroman und 2,1 g 2-Isopropyl-2'-[3(N-methylamino)-propyl]-2"-(3-trifluormethylphenyl)-acetonitril werden in 30 ml absolutem Dimethylformamid gelöst und nach Zugabe von 15 ml Triethylamin 15 Stunden bei 110°C umgesetzt. Nach Abdestillieren des Lösungsmittels wird der Rückstand mit 50 ml 2N Salzsäure verrührt und anschließend bei steigendem pH-Wert mit Diethylether fraktioniert ausgeschüttelt. Die Etherfraktionen bei pH 6 bis pH 7,5 werden vereinigt. Nach dem Trocknen mit Natriumsulfat wird das Lösungsmittel entfernt und das Rohprodukt über Kieselgel (Lichroprep Si 60, Korngröße 15 bis 25 µm; Merck) mit einer Mitteldruckchromatographiesäule nach Helmchen (G. Helmchen et al.; Angew. Chem. 91, 64 (1979)) bei 3 bis 5 bar getrennt.

Man erhält 0,7 g der Verbindung als ein farbloses, viskoses Öl.

$R_f$-Wert: 0,55 ; Elutionsmittel: $CH_2Cl_2/CH_3OH = 9:1$

| Elementaranalyse: | C | H | N |
|---|---|---|---|
| berechnet | 66,65 | 6,99 | 5,55 |
| gefunden | 66,55 | 6,96 | 5,36 |

Beispiel 2

7,8-Dimethoxi-1- {1-[N-(4-cyano-4-(2-fluor-3,4-dimethoxi-phenyl)-5-methyl-hexyl)-N-methyl-amino]-methyl}-1,3,4,5-tetrahydro-2-benzoxepin (Tabelle 1, Verbindung Nr. 6)

2,1 g 1-Brommethyl-7,8-dimethoxi-1,3,4,5-tetrahydro-2-benzoxepin und 2,2 g 2-(3,4-Dimethoxi-2-fluor-phenyl)-2'-isopropyl-2"-[3-(N-methylamino)-propyl]-acetonitril werden in 30 ml absolutem Dimethylformamid gelöst. Anschließend werden 3 g Kaliumcarbonat zugegeben und 15 Stunden bei 110°C gerührt. Nach der in Beispiel 1 beschriebenen Aufarbeitung wird chromatographisch getrennt.

Man erhält 0,2 g der Verbindung als ein gelbliches hochviskoses Öl.

$R_f$-Wert: 0,64; Elutionsmittel: $CH_2Cl_2/CH_3OH$ = 9:1

| Elementaranalyse: | C | H | N |
|---|---|---|---|
| berechnet | 68,16 | 7,82 | 5,30 |
| gefunden | 67,71 | 7,65 | 5,53 |

Beispiel 3

6,7-Dimethoxi-4,4'-dimethyl-1- {2-[N-(4-cyano-4-(3-trifluormethylphenyl)-5-methyl-hexyl)-N-methyl-amino]-ethyl}-isochroman (Tabelle 1, Verbindung Nr. 8)

2 g 2-Bromethyl-6,7-dimethoxi-4,4'-dimethylisochroman und 2,1 g 2-Isopropyl-2'-[3-(N-methylamino)-propyl]-2"-(3-trifluormethylphenyl)-acetonitril werden in 30 ml absolutem Dimethylformamid gelöst. Anschließend werden 3 g Kaliumcarbonat zugegeben und 15 Stunden bei 110°C gerührt. Nach der in Beispiel 1 beschriebenen Aufarbeitung wird chromatographisch getrennt.

Man erhält 1,15 g der Verbindung als ein farbloses, hochviskoses Öl.

$R_f$-Wert: 0,43; Elutionsmittel: $CH_2Cl_2/CH_3OH$ = 9:1

| Elementaranalyse: | C | H | N |
|---|---|---|---|
| berechnet | 68,11 | 7,56 | 5,12 |
| gefunden | 68,04 | 7,67 | 5,37 |

Beispiel 4

6,7-Dimethoxi-1-{2-[N-(4-cyano-4-(4-fluorphenyl)-5-methylhexyl)-N-methyl-amino]-ethyl}-1-methyl-isochroman (Tabelle 1, Verbindung Nr. 10)

8 g 6,7-Dimethoxi-1-methyl-1-[2-(p-tosyloxi)ethyl-isochroman und 5 g 2-(4-Fluorphenyl)-2'-isopropyl-2"-[3-(N-methylamino)-propyl]-acetonitril werden in 50 ml Toluol gelöst und nach Zugabe von 3 ml Triethylamin und 3 g Kaliumcarbonat bei 115°C 15 Stunden gerührt. Nach dem Einengen wird der Rückstand in 100 ml Wasser aufgenommen und mit 100 ml Diethylether extrahiert. Nach dem Abtrennen der Wasserphase wird die organische Phase mit 50 ml 2N Salzsäure versetzt. Die Etherphase wird abgetrennt und die salzsaure Phase bei steigendem pH-Wert mit Diethylether fraktioniert ausgeschüttelt. Zwischen pH 6 und 7,5 werden die getrennten organischen Phasen vereinigt, mit Natriumsulfat getrocknet und anschließend eingeengt.

Daraufhin wird über Kieselgel (Lichroprep Si 60, Korngröße 15 bis 25 µm; Merck) mit einer Mitteldruckchromatographiesäule nach Helmchen (G. Helmchen et al.; Angew. Chem. 91, 64 (1979)) bei 3 bis 5 bar getrennt.

Nach der chromatographischen Aufarbeitung ergaben sich 3,8 g der Verbindung als ein zähes, farbloses Öl.

$R_f$-Wert: 0,32; Elutionsmittel: $CH_2Cl_2/CH_3OH$ = 9:1

| Elementaranalyse: | C | H | N |
|---|---|---|---|
| berechnet | 72,17 | 8,14 | 5,80 |
| gefunden | 71,71 | 8,09 | 5,53 |

Beispiel 5

6,7-Dimethoxi-4,4-dimethyl-1-{2-[N-(4-cyano-4-(3,4-dichlorphenyl)-5-methyl-hexyl)-N-methyl-amino]ethyl}-isochroman (Tabelle 1, Verbindung Nr. 11)

2 g 1-(2-Chlorethyl)-6,7-dimethoxi-4,4'-dimethyl-isochroman werden mit 2,1 g 2-(3,4-Dichlorphenyl)-2'-isopropyl-2"-[3-(N-methylamino)-propyl]-acetonitril in 30 ml Dimethylformamid mit 3 g Kaliumcarbonat 6 Stunden bei 100°C gerührt. Anschließend wird das Reaktionsgemisch eingeengt und analog Beispiel 4 fraktioniert ausgeschüttelt. Die gewünschten Fraktionen werden nach dem Entfernen des Ethers über Kieselgel (Lichroprep SI 60, Merck) mit einer Mitteldruckchromatographiesäule nach Helmchen bei 3 bis 5 bar getrennt. Es fallen 1,1 g reine Substanz als schwach gelbliches, hochviskoses Öl an.

$R_f$-Wert: 0,38; Elutionsmittel: $CH_2Cl_2/CH_3OH$ = 9:1

| Elementaranalyse: | C | H | N |
|---|---|---|---|
| berechnet | 65,80 | 7,36 | 5,12 |
| gefunden | 65,49 | 7,40 | 5,22 |

Beispiel 6

6,7-Dimethoxi-1- {1-[N-(4-cyano-4-(3,4-dichlorphenyl)-5-methyl-hexyl)-N-methyl-amino]methyl} isochroman (Tabelle 1, Verbindung Nr. 14)

2 g 1-Brommethyl-6,7-dimethoxi-isochroman und 2,1 g 2-(3,4-Dichlorphenyl)-2'-isopropyl-2"-[3-(N-methylamino)-propyl]-acetonitril werden in 30 ml absolutem Dimethylformamid gelöst. Nach Zugabe von 3 g Kaliumcarbonat wird 15 Stunden bei 110°C gerührt. Der Reaktionsansatz wird analog Beispiel 4 aufgearbeitet. Es können 0,6 g der Verbindung als zähes, farbloses Öl isoliert werden.

$R_f$-Wert: 0,54; Elutionsmittel: $CH_2Cl_2/CH_3OH = 9:1$

| Elementaranalyse: | C | H | N |
|---|---|---|---|
| berechnet | 64,15 | 6,78 | 5,54 |
| gefunden | 64,03 | 6,77 | 5,44 |

Beispiel 7

7,8-Dimethoxi-1- {1-[N-(4-(3,4-dichlorphenyl)-4-cyano-5-methyl-hexyl)-N-methyl-amino]-methyl} -1,3,4,5-tetrahydro-2-benzoxepin (Tabelle 1, Verbindung Nr. 15)

2,1 g 1-Brommethyl-7,8-dimethoxi-1,3,4,5-tetrahydro-2-benzoxepin und 2,1 g 2-(3,4-Dichlorphenyl)-2'-isopropyl-2"-[3-(N-methylamino)-propyl]-acetonitril werden in 30 ml Dimethyl-formamid gelöst. Nach Zugabe von 3 g Kaliumcarbonat wird 15 Stunden bei 110°C gerührt. Der Reaktionsansatz wird wie im Beispiel 4 beschrieben aufgearbeitet.

Man erhält 0,3 g der Verbindung als ein gelbliches, hochviskoses Öl.

$R_f$-Wert: 0,55; Elutionsmittel: $CH_2Cl_2/CH_3OH$ = 9:1

| Elementaranalyse: | C | H | N |
|---|---|---|---|
| berechnet | 64,73 | 6,98 | 5,39 |
| gefunden | 64,79 | 6,98 | 5,12 |

Beispiel 8

6,7-Dimethoxi-4,4-dimethyl-1-{2-[N-(4-cyano-4-(4-fluorphenyl)-5-methyl-hexyl)-N-methyl-amino]ethyl}-isochroman (Tabelle 1, Verbindung Nr. 19)

2 g 1-(2-Chlorethyl)-6,7-dimethoxi-4,4'-dimethyl-isochroman werden mit 1,8 g 2-(4-Fluorphenyl)-2'-isopropyl-2"-[3-(N-methylamino)-propyl]-acetonitril in 30 ml Dimethylformamid gelöst. Anschließend werden 3 g Kaliumcarbonat zugegeben und 6 Stunden bei 100°C gerührt. Nach dem Abkühlen wird mit Wasser und 2N Salzsäure aufgenommen. Bei steigendem pH-Wert wird mit Diethylether fraktioniert ausgeschüttelt. Die gewünschten Fraktionen werden wie in Beispiel 1 beschrieben chromatographisch aufgearbeitet.

Nach der chromatographischen Trennung konnten 0,8 g reine Verbindung als farbloses Öl isoliert werden.

$R_f$-Wert: 0,52; Elutionsmittel: $CH_2Cl_2/CH_3OH$ = 8:2

Beispiel 9

6,7-Dimethoxi-1{2-[N-(4-cyano-5-methyl-4-(3,4,5-trimethoxiphenyl)hexyl)-N-methyl-amino]ethyl}-isochroman (Tabelle 1, Verbindung Nr. 26)

1,8 g 1-(2-Chlorethyl)-6,7-dimethoxi-isochroman und 2,1 g

2-Isopropyl-2'-[3-(N-methylamino)-propyl]-2"-(3,4,5-trimethoxiphenyl)-acetonitril werden in 30 ml absolutem Dimethylformamid gelöst und nach Zugabe von 10 ml Triethylamin und 3 g Kaliumcarbonat über Nacht bei 100°C gerührt. Nach dem Entfernen des Lösungsmittels im Vakuum wird mit 150 ml Diethylether und mit 100 ml Wasser versetzt und ausgeschüttelt. Die organische Phase wird 3 Mal mit je 80 ml 2N Salzsäure geschüttelt. Die salzsauren wässrigen Phasen werden vereinigt und anschließend mit Soda neutralisiert. Anschließend wird mit 100 ml Diethylether extrahiert. Die organische Phase wird über Natriumsulfat getrocknet und daraufhin eingeengt. Der Rückstand wird über eine Kieselgelsäule chromatographisch getrennt (siehe Beispiel 1).

Man erhält 0,9 g der Verbindung als gelbliches zähes Öl.

<u>$R_f$-Wert:</u> 0,42; <u>Elutionsmittel:</u> $CH_2Cl_2/CH_3CN/C_2H_5OH$ = 7:2:1

| Elementaranalyse: | C | H | N |
|---|---|---|---|
| berechnet | 68,86 | 8,20 | 5,18 |
| gefunden | 68,71 | 8,29 | 4,96 |

Beispiel 10

6,7-Dimethoxi-1{2-[N-(4-Cyano-5-methyl-4(3,4-methylendioxiphenyl)hexyl)-N-methyl-amino]ethyl}-4-methyl-isochroman (Tabelle 1, Verbindung Nr. 28)

1,9 g 1-(2-Chloroethyl)-6,7-dimethoxi-4-methyl-isochroman und 1,9 g 2-Isopropyl-2'-(3,4-methylendioxiphenyl)-2"-[3-(N-methylamino)-propyl]-acetonitril werden in 10 ml absolutem Dimethylformamid gelöst und nach Zugabe von 20 ml Triethylamin und 3 g Kaliumcarbonat 15 Stunden bei 100°C gerührt. Anschließend wird wie in Beispiel 9 beschrieben aufgearbeitet.

Man erhält 250 mg der Verbindung als zähes farbloses Öl.

$R_f$-Wert: 0,41; Elutionsmittel: $CH_2Cl_2/CH_3OH$ = 9:1

| Elementaranalyse: | C | H | N |
|---|---|---|---|
| berechnet | 70,84 | 7,93 | 5,51 |
| gefunden | 70,83 | 8,05 | 5,42 |

Beispiel 11

6,7-Dimethoxi-1-{2-[N-(4-Cyano-5-methyl-4-(3,4,5-trimethoxiphenyl)hexyl)-N-methyl-amino]ethyl}-1,4,4-trimethyl-isochroman (Tabelle 1, Verbindung Nr. 29)

3,4 g 1-(2-Bromethyl)-6,7-dimethoxi-1,4,4'-trimethyl-isochroman und 3,1 g 2-Isopropyl-2'-[3-(N-methylamino)-propyl]-2"-(3,4,5-trimethoxiphenyl)-acetonitril werden in 15 ml Dimethylformamid gelöst und nach Zugabe von 30 ml Triethylamin und 3 g Kaliumcarbonat 12 Stunden bei 120°C gerührt. Anschließend wird analog Beispiel 1 fraktioniert ausgeschüttelt. Die gewünschten Fraktionen (pH 6 - pH 7,5) werden vereinigt. Die Etherphase wird getrocknet und anschließend entfernt. Das Rohprodukt wird chromatographisch gereinigt.

Nach dem chromatographischen Aufarbeiten können 1,9 g der Verbindung als leicht gelbliches zähes Öl erhalten werden.

$R_f$-Wert: 0,34; Elutionsmittel: $CH_2Cl_2/CH_3OH$ = 9:1

| Elementaranalyse: | C | H | N |
|---|---|---|---|
| berechnet | 70,07 | 8,65 | 4,81 |
| gefunden | 69,70 | 8,90 | 4,67 |

Beispiel 12

6,7-Dimethoxi-1- {2-[N-(4-Cyano-4-(4-fluorphenyl)-5-methylhexyl)-N-methyl-amino]-ethyl}-1,4,4-trimethyl-isochroman (Tabelle 1, Verbindung Nr. 31)

3,4 g 1-(2-Bromethyl)-6,7-dimethoxi-1,4,4'-trimethyl)-isochroman, 2,5 g 2-(4-Fluorphenyl)-2'-isopropyl-2"-[3-(N-methyl-amino)-propyl]-acetonitril, 20 ml Triethylamin und 3 g Kaliumcarbonat werden entsprechend Beispiel 1 umgesetzt und aufgearbeitet. Nach der chromatographischen Aufarbeitung konnten 1,8 g reines Produkt als farbloses, hochviskoses Öl erhalten werden.

$R_f$-Wert: 0,36; Elutionsmittel: $CH_2Cl_2/CH_3OH = 9:1$

Beispiel 13

6,7-Dimethoxi-1- {2-[N-(4-Cyano-5-methyl-4-(3-trifluormethylphenyl)hexyl)-N-methyl-amino]ethyl}-isochroman (Tabelle 1, Verbindung Nr. 33)

1,8 g 1-(2-Chloroethyl)-6,7-dimethoxi-isochroman, 2,1 g 2-Isopropyl-2'-[3-(N-methylamino)-propyl]-2"-(3-trifluorphenyl)-acetonitril, 10 ml Dimethylformamid, 20 ml Triethylamin und 3 g Kaliumcarbonat werden analog Beispiel 1 umgesetzt und aufgearbeitet.
Nach der chromatographischen Trennung konnten 0,8 g reines Produkt als farbloses, zähes Öl isoliert werden.

$R_f$-Wert: 0,42; Elutionsmittel: $CH_2Cl_2/CH_3OH = 9:1$

| Elementaranalyse: | C | H | N |
|---|---|---|---|
| berechnet | 67,16 | 7,19 | 5,40 |
| gefunden | 66,82 | 7,22 | 5,15 |

Beispiel 14
6,7-Dimethoxi-1- {2-[N-(4-Cyano-4-(3,4-dimethoxiphenyl)-5-methyl-hexyl)-N-methyl-amino]ethyl}-1-methyl-isochroman
(Tabelle 1, Verbindung Nr. 35)

4 g 6,7-Dimethoxi-1-methyl-1-[2-(p-tosyloxi)-ethyl]-isochroman und 2,9 g 2-(3,4-Dimethoxiphenyl)-2'-isopropyl-2"-[3-(N-methylamino)-propyl]-acetonitril werden in 30 ml trockenem Toluol und 5 ml Dimethylformamid gelöst und nach Zugabe von 5 ml Triethylamin und 3 g Kaliumcarbonat 15 Stunden bei 100°C gerührt. Die Aufarbeitung erfolgt analog Beispiel 4. Nach der chromatographischen Trennung über Kieselgel erhält man 3,1 g der Verbindung als hochviskoses, farbloses Öl.

$R_f$-Wert: 0,21; Elutionsmittel: $CH_2Cl_2OCH_3OH$ = 9:1

| Elementaranalyse: | C | H | N |
|---|---|---|---|
| berechnet | 70,96 | 8,45 | 5,34 |
| gefunden | 70,47 | 8,49 | 5,14 |

Pharmazeutische Formulierungsbeispiele

a) Dragées

1 Dragéekern enthält:

| | |
|---|---|
| Wirkstoff gemäß vorliegender Erfindung | 2,0 mg |
| Milchzucker | 28,5 mg |
| Maisstärke | 17,0 mg |
| Gelatine | 2,0 mg |
| Magnesiumstearat | 0,5 mg |
| | 50,0 mg |

Herstellung:
Die Mischung der Wirksubstanz mit Milchzucker und Maisstärke wird mit einer 10%igen wässrigen Gelatinelösung durch ein Sieb mit 1 mm Maschenweite granuliert, bei 40°C getrocknet und nochmals durch ein Sieb getrieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxid, Talkum und Gummi arabicum aufgebracht wird. Die fertigen Dragées werden mit Bienenwachs poliert. Dragée-Endgewicht: 100 mg.

b) Tabletten

| | |
|---|---|
| Wirkstoff gemäß vorliegender Erfindung | 2,0 mg |
| Milchzucker | 55,0 mg |
| Maisstärke | 38,0 mg |
| lösliche Stärke | 4,0 mg |
| Magnesiumstearat | 1,0 mg |
| | 100,0 mg |

Herstellung:
Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 100 mg Gewicht verpreßt, die 2 mg Wirkstoff enthalten.

c) Suppositorien

1 Zäpfchen enthält:

| | |
|---|---|
| Wirkstoff gemäß vorliegender Erfindung | 1,0 mg |
| Zäpfchenmasse | 1699,0 mg |

Herstellung:
Die feingepulverte Substanz wird mit Hilfe eines Eintauchhomogenisators in die geschmolzene und auf 40°C abgekühlte Zäpfchenmasse eingerührt. Die Masse wird bei 35°C in leicht vorgekühlte Formen gegossen.

d) Ampullen

| | | |
|---|---|---|
| Wirkstoff gemäß vorliegender Erfindung | | 2,0 mg |
| Natriumchlorid | | 18,0 mg |
| destilliertes Wasser | ad | 2,0 ml |

Herstellung:
Wirkstoff und Natriumchlorid werden in Wasser gelöst, die Lösung frei von suspendierten Partikeln filtriert und in 2 ccm-Ampullen unter aseptischen Bedingungen abgefüllt. Zuletzt werden die Ampullen sterilisiert und verschlossen. Jede Ampulle enthält 2 mg Wirkstoff.

## PATENTANSPRÜCHE:

1. Substituierte Isochromane und Oxepine der allgemeinen Formel (I)

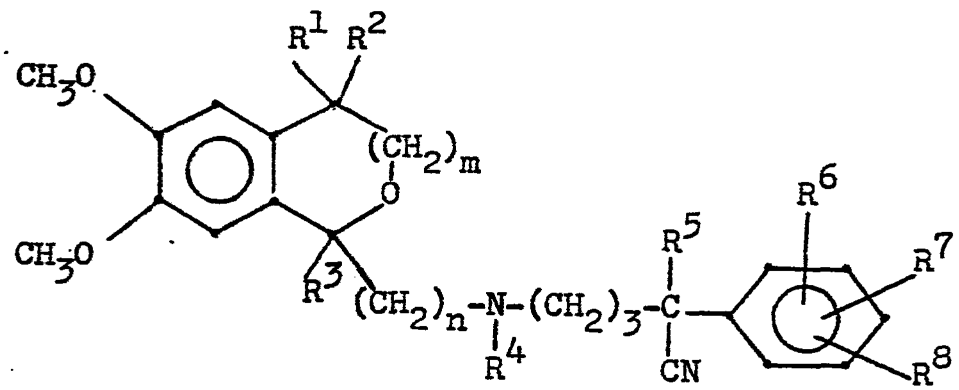

I

worin

$R^1$ ein Wasserstoffatom oder eine gerade oder verzweigte Alkylgruppe mit bis zu 4 C-Atomen,

$R^2$ ein Wasserstoffatom oder eine gerade oder verzweigte Alkylgruppe mit bis zu 4 C-Atomen oder zusammen mit $R^1$ eine Alkylengruppe mit 3 bis 5 C-Atomen,

$R^3$ ein Wasserstoffatom oder eine gerade oder verzweigte Alkylgruppe mit bis zu 4 C-Atomen,

$R^4$ ein Wasserstoffatom oder eine gerade oder verzweigte Alkylgruppe mit bis zu 4 C-Atomen,

$R^5$ ein Wasserstoffatom oder eine gerade oder verzweigte Alkylgruppe mit 1 bis 6 C-Atomen oder eine Pyridinylgruppe,

$R^6$ ein Wasserstoffatom, ein Fluor-, Chlor- oder Bromatom, eine gerade oder verzweigte Alkylgruppe mit 1 bis 6 C-Atomen, eine Hydroxi- oder Alkylgruppe mit 1 bis 4 C-Atomen oder eine Trifluormethylgruppe,

$R^7$ ein Wasserstoffatom, ein Fluor-, Chlor- oder Bromatom, eine gerade oder verzweigte Alkylgruppe mit 1 bis 6

C-Atomen, eine Hydroxi- oder Alkoxigruppe mit 1 bis 4 C-Atomen oder zusammen mit $R^6$ eine Methylendioxigruppe,

$R^8$ ein Wasserstoffatom, ein Fluor-, Chlor- oder Bromatom, eine Hydroxi- oder Alkoxigruppe mit 1 bis 4 C-Atomen,

m die Zahlen 1 oder 2 und

n die Zahlen 1, 2 oder 3 bedeuten

sowie deren Säureadditionssalze.

2. Verbindungen nach Anspruch 1 der allgemeinen Formel (Ia)

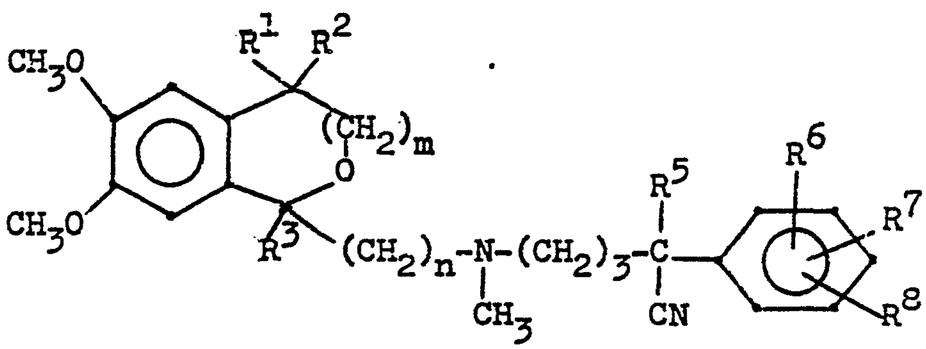

Ia

worin $R^1$, $R^2$ und $R^3$, die gleich oder verschieden sein können, ein Wasserstoffatom oder eine Methylgruppe

$R^5$ eine Isopropyl- oder Pyridinylgruppe,

$R^6$ ein Wasserstoff-, Fluor- oder Chloratom, eine Trifluormethyl- oder Methoxigruppe,

$R^7$ ein Wasserstoff-, Fluor- oder Chloratom, eine tertiäre Butylgruppe, eine Methoxigruppe oder zusammen mit $R^6$ eine Methylendioxigruppe,

$R^8$ ein Wasserstoffatom oder eine Methoxigruppe,

m die Zahlen 1 oder 2 und

n die Zahlen 1 oder 2 bedeuten

sowie deren Säureadditionssalze.

3. Verbindungen nach Anspruch 1 der allgemeinen Formel (Ib)

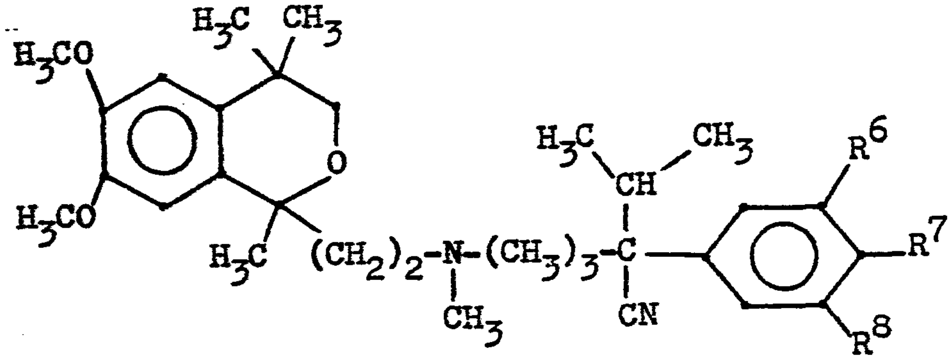

Ib

worin
$R^6$ ein Wasserstoff- oder Chloratom oder eine Methoxigruppe,
$R^7$ ein Fluor- oder Chloratom oder eine Methoxigruppe und
$R^8$ ein Wasserstoffatom oder eine Methoxigruppe bedeuten,
sowie deren Säureadditionssalze.

4. Verfahren zur Herstellung von Verbindungen nach Anspruch 1, dadurch gekennzeichnet, daß man ein Isochroman- oder Oxepinderivat der allgemeinen Formel (II)

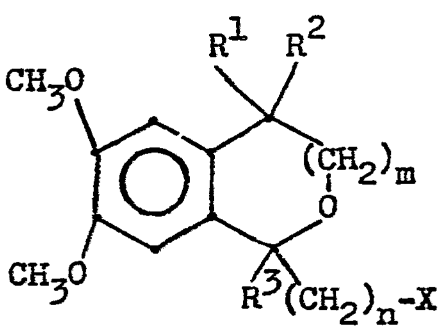

II

worin $R^1$, $R^2$, $R^3$, m und n wie oben angegeben definiert

sind und X ein Chlor-, Brom- oder Jodatom oder eine Alkylsulfonyloxi- oder Arylsulfonyloxigruppe bedeutet, mit einem Phenyl-aminopropyl-acetonitril der allgemeinen Formel (III)

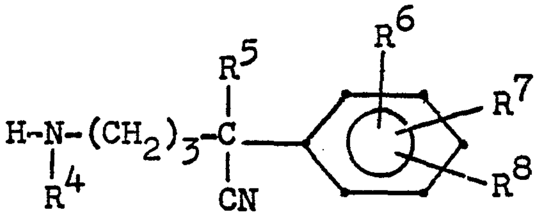

III

worin $R^4$, $R^5$, $R^6$, $R^7$ und $R^8$ die oben angegebene Bedeutung besitzen, umsetzt und die erhaltene Verbindung gewünschtenfalls in ein Säureadditionssalz überführt.

5. Verfahren nach Anspruch 4, dadurch gekennzeichnet, daß man die Umsetzung in Gegenwart eines säurebindenden Mittels bei Temperaturen von 50 bis 160°C in einem aprotischen Lösungsmittel durchführt.

6. Pharmazeutische Zubereitungen, dadurch gekennzeichnet, daß sie eine oder mehrere Verbindungen nach Anspruch 1 neben üblichen Hilfs- und Trägerstoffen enthalten.

7. Verfahren zur Herstellung von pharmazeutischen Zubereitungen nach Anspruch 6, dadurch gekennzeichnet, daß man Verbindungen nach Anspruch 1 mit üblichen galenischen Hilfs- und Trägerstoffen zu üblichen pharmazeutischen Darreichungsformen verarbeitet.

8. Verbindungen nach Anspruch 1 zur Verwendung für die Herstellung von Arzneimitteln mit calciumantagonistischer und antihypertonischer Wirkung.